# EUROPEAN PATENT SPECIFICATION

(11) **EP 4 012 880 B1**
(45) Date of publication and mention of the grant of the patent: **27.12.2023**
(21) Application number: 20849615.8
(22) Date of filing: 31.07.2020
(51) Int. Cl.: H02J 7/00, G01R 31/3835, A61B 18/12

(54) **CHARGING AND DISCHARGING MONITORING CIRCUIT AND TUMOR THERAPY INSTRUMENT**
LADE- UND ENTLADEÜBERWACHUNGSSCHALTUNG UND TUMORTHERAPIEGERÄT
CIRCUIT DE SURVEILLANCE DE CHARGE ET DE DÉCHARGE ET INSTRUMENT DE THÉRAPIE ANTITUMORALE

(30) Priority: 06.08.2019 CN 201910720954
(43) Date of publication of application: 15.06.2022
(73) Proprietor: Shenzhen Neumann Technology Co., Ltd., Shenzhen, Guangdong 518000 (CN)
(72) Inventor: LAI, Shen, Shenzhen, Guangdong 518000 (CN)
(74) Representative: Marks & Clerk LLP
(86) International application number: PCT/CN2020/106410
(87) International publication number: WO 2021/023128

(56) References cited:
- CN-A- 101 618 253
- CN-A- 105 429 226
- CN-A- 109 787 322
- CN-U- 207 926 262
- CN-U- 208 369 238
- GB-A- 2 489 498
- JP-A- 2016 088 178
- JP-B2- 6 397 720
- TW-B- I 551 000
- US-A1- 2010 134 305
- US-A1- 2017 070 073
- US-A1- 2017 264 123
- US-B2- 9 917 464

## Description

### TECHNICAL FIELD

The present application relates to the field of medical device technique, and particularly to a charge and discharge monitoring circuit and a tumor therapy instrument.

### BACKGROUND

With the development of science and technology, the application of tumor therapy instruments has received wide attention. There are many types of tumor therapy instruments. The working process of the tumor therapy instrument based on an electric pulse technology is generally as follows: the instantaneous electric field intensity of the pulsed electric field applied to the cell is greater than 1kV/cm (kilovolts per centimeter), accordingly the molecular permeability of the cell is greatly increased, and then the electroporation is produced. As the pulsed electric field intensity continues to increase, irreversible electrical breakdown occurs, which leads to mechanical breakage of the cell membrane until the cell death. The tumor therapy instrument has strict requirements for output pulses, and the stability of the charge and discharge performance of the tumor therapy instrument directly affects the reliability of the pulse output. Therefore, the monitoring of the charge and discharge of the tumor therapy instrument has great significance.

In the implementation process, the inventors found that the conventional technology at least has the following problems: the design of the conventional circuit based on the charge and discharge monitoring of the tumor therapy instrument is complicated, and the charge and discharge monitoring delay is large.

### SUMMARY

In view of this, as for the problems of complicated design of the conventional circuit based on the charge and discharge monitoring of the tumor therapy instrument and the large delay in the charge and discharge monitoring, it is necessary to provide a charge and discharge monitoring circuit and a tumor therapy instrument.

In order to achieve the above purpose, according to an embodiment of the present invention, a charge and discharge monitoring circuit is provided, including a capacitor bank and a power supply configured to power the capacitor bank; further including:
a first switch group, a first switch interface of the first switch group being connected to a positive electrode of the power supply, and a second switch interface of the first switch group being connected to a positive electrode of the capacitor bank;
a second switch group, a first switch interface of the second switch group being connected to a negative electrode of the power supply, and a second switch interface of the second switch group being connected to a negative electrode of the capacitor bank;
a discharge module, an input terminal of the discharge module being connected to the positive electrode of the capacitor bank, and an output terminal of the discharge module being connected to the negative electrode of the capacitor bank;
a voltage detection circuit, a first detection terminal of the voltage detection circuit being connected to a third switch interface of the first switch group, and a second detection terminal of the voltage detection circuit being connected to a third switch interface of the second switch group, the voltage detection circuit being configured to monitor a charge voltage signal of the capacitor bank and monitor a discharge voltage signal of the discharge module;
a controller, the controller being respectively connected to an output terminal of the voltage detection circuit, a control interface of the first switch group, a control interface of the second switch group and a control terminal of the discharge module; the controller being configured to, when receiving a charge monitoring instruction, control the discharge module to turn off, and control the first switch group and the second switch group to turn on, and acquire a charge voltage signal to obtain a charge voltage; the controller being configured to, when receiving a discharge monitoring instruction, control the discharge module to turn on, and control the first switch group and the second switch group to turn off, and acquire a discharge voltage signal to obtain a discharge voltage.

In an embodiment, the discharge module includes a first switch device and a discharge load;
a first end of the first switch device is connected to a first end of the discharge load, a second end of the first switch device is connected to one end of the capacitor bank, and a third end of the first switch device is connected to the controller, a second end of the discharge load is connected to the other end of the capacitor bank.

In an embodiment, the first switch group includes at least two second switch devices connected in series.

In an embodiment, the second switch group includes at least two third switch devices connected in series.

In an embodiment, the charge and discharge monitoring circuit further includes a first isolation module;
a first input terminal of the first isolation module is connected to the first switch group, a second input terminal of the first isolation module is connected to the second switch group, and an output terminal of the first isolation module is connected to the voltage detection circuit.

In an embodiment, the charge and discharge monitoring circuit further includes a second isolation module;
the second isolation module is connected between the voltage detection circuit and the controller.

In an embodiment, the voltage detection circuit includes a voltage divider circuit;
a first detection terminal of the voltage divider circuit is connected to the third switch interface of the first switch group, and a second detection terminal of the voltage divider circuit is connected to the third switch interface of the second switch group, and an output terminal of the voltage divider circuit is connected to the controller.

In an embodiment, the controller includes a processing chip and an AD acquisition circuit connected to the processing chip.

In another aspect, according to an embodiment of the present invention, a tumor therapy instrument is provided, which includes an upper computer and the charge and discharge monitoring circuit according to any one of the above embodiments;
the upper computer is connected to the controller.

In an embodiment, the tumor therapy instrument further includes an alarm device connected to the controller.

One of the above technical solutions has the following advantages and beneficial effects.

The first switch group is connected between the positive electrode of the power supply and the positive electrode of the capacitor bank; the second switch group is connected between the negative electrode of the power supply and the negative electrode of the capacitor bank. The discharge module is connected between the positive electrode of the capacitor bank and the negative electrode of the capacitor bank. The first detection terminal of the voltage detection circuit is connected to the third switch interface of the first switch group, and the second detection terminal is connected to the third switch interface of the second switch group; the controller, when receiving the charge monitoring instruction, controls the discharge module to turn off, and controls the first switch group and the second switch group to turn on, and acquires the charge voltage signal to obtain the charge voltage; the controller, when receiving the discharge monitoring instruction, controls the discharge module to turn on, and controls the first switch group and the second switch group to turn off, and acquires the discharge voltage signal to obtain the discharge voltage, thereby implementing the real-time charge and discharge monitoring of the tumor therapy instrument. As for the real-time charge and discharge monitoring circuit in each embodiment of the present invention, the circuit structure is simple, and the real-time monitoring of the charge and discharge is improved.

### BRIEF DESCRIPTION OF THE DRAWINGS

FIG 1 is a schematic structure diagram I of a charge and discharge monitoring circuit according to an embodiment of the present invention.
FIG 2 is a schematic structure diagram II of a charge and discharge monitoring circuit according to an embodiment of the present invention.
FIG 3 is a schematic structure diagram III of a charge and discharge monitoring circuit according to an embodiment of the present invention.
FIG 4 is a schematic structure diagram IV of a charge and discharge monitoring circuit according to an embodiment of the present invention.
FIG 5 is a schematic structure diagram V of a charge and discharge monitoring circuit according to an embodiment of the present invention.
FIG 6 is a schematic block diagram illustrating a tumor therapy instrument according to an embodiment of the present invention.

### DETAILED DESCRIPTION

In order to facilitate the understanding of the present application, the application will be described in a more comprehensive manner with reference to the relevant accompanying drawings. Preferred embodiments of the present invention are shown in the accompanying drawings. However, the present application can be implemented in many different forms and is not limited to the embodiments described herein. Rather, the purpose of providing these embodiments is to make the disclosure of the present application more thorough and comprehensive.

Unless otherwise defined, all technical and scientific terms used herein have the same meaning as commonly understood by those skilled in the technical field of the present application. The terms used in the description of the present application herein are merely for the purpose of describing the specific embodiments, and are not intended to limit the present application. The term "and/or" as used herein includes any and all combinations of one or more related listed items.

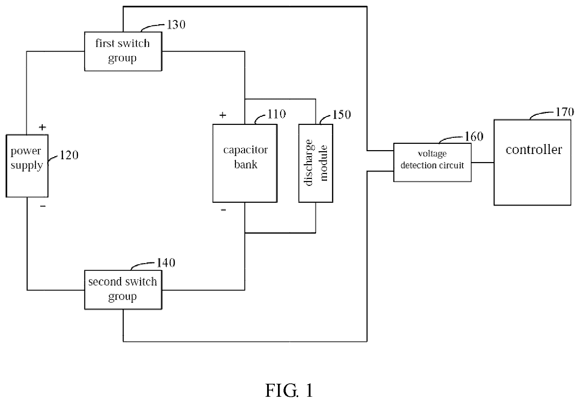

In order to solve the problem of the complicated design of the conventional circuit based on the charge and discharge monitoring of the tumor therapy instrument and the large delay in the charge and discharge monitoring, according to an embodiment, as shown in FIG 1, a charge and discharge monitoring circuit is provided, which includes a capacitor bank 110 and a power supply 120 configured to power the capacitor bank 110; and further includes:
a first switch group 130; a first switch interface of the first switch group 130 is connected to a positive electrode of the power supply 120, and a second switch interface of the first switch group 130 is connected to a positive electrode of the capacitor bank 110;
a second switch group 140; a first switch interface of the second switch group 140 is connected to a negative electrode of the power supply 120, and a second switch interface of the second switch group 140 is connected to a negative electrode of the capacitor bank 110;
a discharge module 150; an input terminal of the discharge module 150 is connected to the positive electrode of the capacitor bank 110, and an output terminal of the discharge module 150 is connected to the negative electrode of the capacitor bank 110;
a voltage detection circuit 160; a first detection terminal of the voltage detection circuit 160 is connected to a third switch interface of the first switch group 130, and a second detection terminal of the voltage detection circuit 160 is connected to a third switch interface of the second switch group 140; the voltage detection circuit 160 is configured to monitor a charge voltage signal of the capacitor bank 110, and monitor a discharge voltage signal of the discharge module 150;
a controller 170; the controller 170 is respectively connected to an output terminal of the voltage detection circuit 160, a control interface of the first switch group 130, a control interface of the second switch group 140, and a control terminal of the discharge module 150; the controller 170, when receiving a charge monitoring instruction, controls the discharge module 150 to turn off, and controls the first switch group 130 and the second switch group 140 to turn on, and acquires a charge voltage signal to obtain a charge voltage; the controller 170, when receiving a discharge monitoring instruction, controls the discharge module 150 to turn on, and controls the first switch group 130 and the second switch group 140 to turn off, and acquires a discharge voltage signal to obtain a discharge voltage.

The capacitor bank 110 can be configured to store electrical energy, and transmit the stored electrical energy to a pulse generator, so that the pulse generator generates an electrical pulse signal. The capacitor bank 110 can consist of a plurality of capacitors in parallel, or the capacitor bank 110 can also consist of a plurality of capacitors in series. The power supply 120 can be a DC high-voltage power supply, for example, the power supply 120 can be a kilovolt-level high-voltage power supply. The first switch group 130 can be configured to control on-off between the positive electrode of the power supply 120 and the positive electrode of the capacitor bank 110. The second switch group 140 can be configured to control the on-off between the negative electrode of the power supply 120 and the negative electrode of the capacitor bank 110. The discharge module 150 can be configured to discharge the electric energy stored in the capacitor bank 110 and can also be configured to control the on-off of a discharge path of the capacitor bank 110. The voltage detection circuit 160 can be configured to detect voltages of power supply signals inputted into both ends of the capacitor bank 110. For example, the voltage detection circuit 160 can acquire the power supply signal, and perform conversion processing (for example, filtering and/or voltage division) on the acquired power supply signal, and then can obtain the charge voltage signal corresponding to the power supply signal. The voltage detection circuit 160 can also be configured to detect voltages of the discharge signals at both ends of the discharge module 150. For example, the voltage detection circuit 160 can acquire the discharge signal, and perform conversion processing (for example, filtering and/or voltage division) on the acquired discharge signal, and then can obtain the discharge voltage signal corresponding to the discharge signal. The controller 170 refers to a processing device with functions such as signal transmission, and signal acquisition and processing.

The charge voltage signal refers to an analog charge voltage signal; and the charge voltage refers to a digital charge voltage. The discharge voltage signal refers to an analog discharge voltage signal; and the discharge voltage refers to a digital discharge voltage.

It should be noted that the charge monitoring instruction can be generated based on a user request, or can be automatically generated when the system determines that a charge monitoring condition is satisfied. The discharge detection instruction can be generated based on a user request, or automatically generated when the system determines that a discharge monitoring condition is satisfied.

Specifically, the first switch group 130 is connected between the positive electrode of the power supply 120 and the positive electrode of the capacitor bank 110; the second switch group 130 is connected between the negative electrode of the power supply 120 and the negative electrode of the capacitor bank 110; and the discharge module 150 is connected between the positive electrode of the capacitor bank 110 and the negative electrode of the capacitor bank 110; the controller 170 is connected to the first switch group 130, the second switch group 140, and the discharge module 150 respectively, and the controller 170 can respectively control the on-off of the first switch group 130, the second switch group 140 and the discharge module 150 according to the received monitoring instruction (such as the charge monitoring instruction and the discharge monitoring instruction), thereby implementing the switching between the charge monitoring path and the discharge monitoring path of the tumor therapy instrument, and simplifying the charge and discharge monitoring circuit.

The first detection terminal of the voltage detection circuit 160 is connected to the third switch interface of the first switch group 130, and the second detection terminal is connected to the third switch interface of the second switch group 140; the controller 170 is connected to the voltage detection circuit 160 to control, when receiving the charge monitoring instruction, the discharge module 150 to turn off and control the first switch group 130 and the second switch group 140 to turn on, and acquire the charge voltage signal to obtain the charge voltage; the controller 170, when receiving the discharge monitoring instruction, controls the discharge module 150 to turn on, and controls the first switch group 130 and the second switch group 140 to turn off, and the acquires the discharge voltage signal to obtain the discharge voltage, thereby implementing the real-time charge and discharge monitoring of the tumor therapy instrument.

In the above-mentioned rea-time charge and discharge monitoring circuit, the on-off of the first switch group, the second switch group and the discharge module are respectively controlled by the controller, to implement the switching between the charge monitoring path and the discharge monitoring path of the tumor therapy instrument, and simplify the charge and discharge monitoring circuit. The charge voltage signal and the discharge voltage signal are acquired by the controller, and the acquired charge voltage signal and discharge voltage signal are processed by the controller, thereby implementing the real-time monitoring of the charge voltage and charge current of the tumor therapy instrument, and improving the real-time monitoring of the charge and discharge.

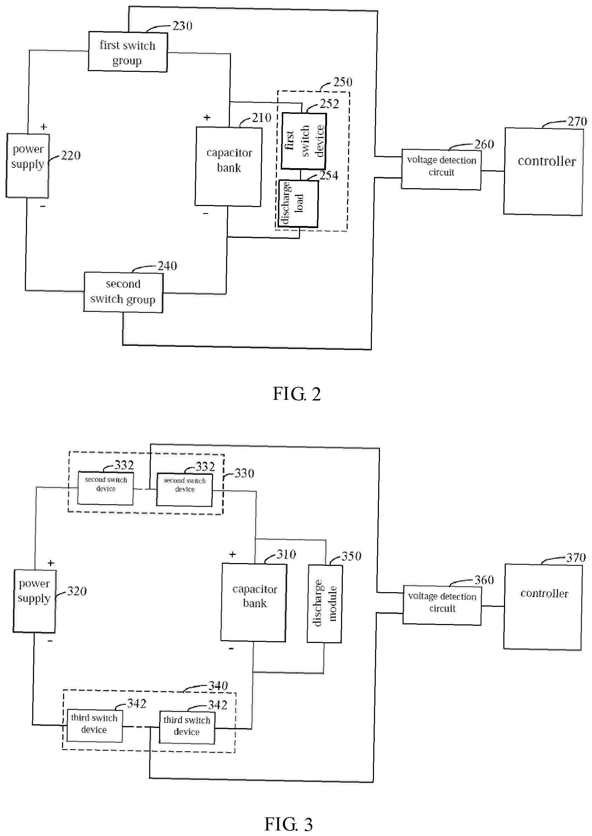

In an embodiment, as shown in FIG 2, a charge and discharge monitoring circuit is provided, which includes a controller 270, a capacitor bank 210, a power supply 220 configured to power the capacitor bank, a first switch group 230 connected between a positive electrode of the power supply 220 and a positive electrode of the capacitor bank 210, a second switch group 240 connected between a negative electrode of the power supply 220 and a negative electrode of the capacitor bank 210, a discharge module 250 connected between the positive electrode of the capacitor bank 210 and the negative electrode of the capacitor bank 210, and a voltage detection circuit 260 connected between the first switch group 230 and the second switch group 240. The controller 270 is respectively connected to the voltage detection circuit 260, the first switch group 230, the second switch group 240, and the discharge module 250.

The discharge module 250 includes a first switch device 252 and a discharge load 254. A first end of the first switch device 252 is connected to a first end of the discharge load 254, a second end of the first switch device 252 is connected to one end of the capacitor bank 210, and a third end of the first switch device 252 is connected to the controller 270; a second end of the discharge load 254 is connected to the other end of the capacitor bank 210.

Specifically, the first switch device 252 refers to an electronic element that can turn off a circuit, interrupt a current, or make the current flow to other circuits. The first switch device 252 may be, but is not limited to, a MOSFET switch device and an IGBT switch. The discharge load 254 can be configured to consume the electric energy of the capacitor bank.

The first end of the first switch device 252 is connected to the first end of the discharge load 254, the second end of the first switch device 252 is connected to one end of the capacitor bank 210, and the third end of the first switch device 252 is connected to the controller 270, and the second end of the discharge load 254 is connected to the other end of the capacitor bank 210. The controller 270 can control the on-off of the discharge channel of the capacitor bank through the on-off of the first switch device 252; and then the controller 270, when receiving the charge monitoring instruction, controls the first switch device 252 to turn off and controls the first switch group 230 and the second switch group 240 to turn on, and acquires the charge voltage signal to obtain the charge voltage, thereby implementing the real-time monitoring of the charge voltage. The controller 270, when receiving the discharge monitoring instruction, controls the first switch device 252 to turn on, and controls the first switch group 230 and the second switch group 240 to turn off, and acquires the discharge voltage signal to obtain the discharge voltage, thereby implementing the real-time monitoring of the discharge voltage.

In an embodiment, as shown in FIG 3, a charge and discharge monitoring circuit is provided, which includes a controller 370, a capacitor bank 310, a power supply 320 configured to power the capacitor bank 310, a first switch group 330 connected between a positive electrode of the power supply 320 and a positive electrode of the capacitor bank 310, a second switch group 340 connected between a negative electrode of the power supply 320 and a negative electrode of the capacitor bank 310, a discharge module 350 connected between the positive electrode of the capacitor bank 310 and the negative electrode of the capacitor bank 310, a voltage detection circuit 360 connected between the first switch group 330 and the second switch group 340; the controller 370 is respectively connected to the voltage detection circuit 360, the first switch group 330, the second switch group 340 and the discharge module 350. The first switch group 330 includes at least two second switch devices 332 connected in series.

Specifically, the second switching device 332 refers to an electronic element that can turn off a circuit, interrupt a current, or make the current flow to other circuits. The second switch device 332 may be, but is not limited to, a MOSFET switch device and an IGBT switch.

For example, the first switch group 330 includes two second switch devices 332 connected in series; and a first detection terminal of the voltage detection circuit 360 is connected between the two second switch devices 332 connected in series. Furthermore, the controller 370, when receiving the charge monitoring instruction, controls the discharge module 350 to turn off, and controls the second switch group 340 to turn on, and controls the two second switch devices 332 connected in series to turn on, and acquires the charge voltage signal to obtain the charge voltage, thereby implementing the real-time monitoring of the charge voltage. The controller 370, when receiving the discharge monitoring instruction, controls the discharge module 350 to turn on, and controls the second switch group 340 to turn off, and controls the two second switch devices 332 connected in series to turn off, and acquires the discharge voltage signal to obtain the discharge voltage, thereby implementing the real-time monitoring of the discharge voltage.

It should be noted that the first detection terminal of the voltage detection circuit can also be connected to a connection line between the second switch device and the power supply.

In a specific embodiment, as shown in FIG 3, the second switch group 340 includes at least two third switch devices 342 connected in series.

Specifically, the third switch device 342 refers to an electronic element that can turn off a circuit, interrupt a current, or make the current flow to other circuits. The third switch device 342 may be, but is not limited to, a MOSFET switch device and an IGBT switch.

For example, the second switch group 340 includes two third switch devices 342 connected in series; the second detection terminal of the voltage detection circuit 360 is connected between the two third switch devices 342 connected in series. Furthermore, the controller 370, when receiving the charge monitoring instruction, controls the discharge module 350 to turn off, and controls the first switch group 330 to turn on, and controls the two third switch devices 342 connected in series to turn on, and acquires the charge voltage signal to obtain the charge voltage, thereby implementing the real-time monitoring of the charge voltage. The controller 370, when receiving the discharge monitoring instruction, controls the discharge module 350 to turn on, and controls the first switch group 330 to turn off, and controls the two third switch devices 342 connected in series to turn off, and acquires the discharge voltage signal to obtain the discharge voltage, thereby implementing the real-time monitoring of the discharge voltage.

It should be noted that the second detection terminal of the voltage detection circuit can also be connected to the connection line between the third switch device and the power supply.

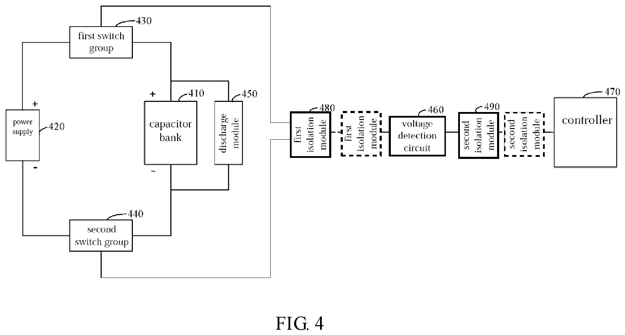

In an embodiment, as shown in FIG 4, a charge and discharge monitoring circuit is provided, which includes a controller 470, a capacitor bank 410, a power supply 420 configured to power the capacitor bank 410, a first switch group 410 connected between a positive electrode of the power supply 420 and a positive electrode of the capacitor bank 410, a second switch group 440 connected between a negative electrode of the power supply 420 and a negative electrode of the capacitor bank 410, a discharge module 450 connected between the positive electrode of the capacitor bank 410 and the negative electrode of the capacitor bank 410, a voltage detection circuit 460 connected between the first switch group 430 and the second switch group 440; the controller 470 is respectively connected to the voltage detection circuit 460, the first switch group 430, the second switch group 440 and the discharge module 450. The charge and discharge monitoring circuit 460 further includes a first isolation module 480. A first input terminal of the first isolation module 480 is connected to the first switch group 430, a second input terminal of the first isolation module 480 is connected to the second switch group 440, and an output terminal of the first isolation module 480 is connected to the voltage detection circuit 460.

The first isolation module 480 can be, but is not limited to, an outer optical path photocoupler and an inner optical path photocoupler.

Specifically, the first isolation module 480 is connected between the voltage detection circuit 460 and the switch group (the first switch group 430 and the second switch group 440); the power supply 420 can transmit the outputted power supply signal to the first isolation module 480 during the charging; the power supply signal is isolated and converted through the first isolation module 480, and the processed power supply signal is transmitted to the voltage detection circuit 460, such that the voltage detection circuit 460 obtains the charge voltage signal according to the power supply signal, thereby implementing the isolation of the power supply signal from the charge voltage signal. In the process of discharging the capacitor bank 410 to the discharge module 450, the outputted discharge signal can be transmitted to the first isolation module 480 to isolate and convert the discharge signal through the first isolation module 480, and the processed discharge signal is transmitted to the voltage detection circuit 460, such that the voltage detection circuit 460 obtains the discharge voltage signal according to the discharge signal, thereby implementing the isolation of the discharge signal from the discharge voltage signal, and improving the anti-interference capability of the signal transmission on the switch group side.

It should be noted that, as shown in FIG 4, the number of the first isolation modules is at least one. For example, when the charge and discharge monitoring circuit includes two or more second isolation modules 480, each second isolation module 480 is connected in series between the voltage detection circuit 460 and the switch group (the first switch group 430 and the second switch group 440).

In an embodiment, as shown in FIG 4, the charge and discharge monitoring circuit further includes a second isolation module 490; the second isolation module 490 is connected between the voltage detection circuit 460 and the controller 470.

The second isolation module 490 may be, but is not limited to, an outer optical path photocoupler and an inner optical path photocoupler.

Specifically, the second isolation module 490 is connected between the voltage detection circuit 460 and the controller 470; in the signal acquisition process of the controller 470, the voltage signal (the charge voltage signal or discharge voltage signal) outputted from the voltage detection circuit 460 can be transmitted to the second isolation module 490 to isolate and convert the voltage signal (the charge voltage signal or discharge voltage signal) through the second isolation module 490, and the processed voltage signal (the charge voltage signal or discharge voltage signal) is transmitted to the controller 470, accordingly the corresponding charge voltage or discharge voltage can be obtained according to the acquired voltage signal (the charge voltage signal or discharge voltage signal), thereby implementing the isolation of the charge voltage signal from the charge voltage (or the isolation of the discharge voltage signal from the discharge voltage), and improving the anti-interference capability of the signal transmission on the controller side.

It should be noted that, as shown in FIG 4, the number of the second isolation modules 490 is at least one. For example, when the charge and discharge monitoring circuit includes two or more second isolation modules 490, each second isolation module 490 is connected in series between the voltage detection circuit 460 and the controller 470.

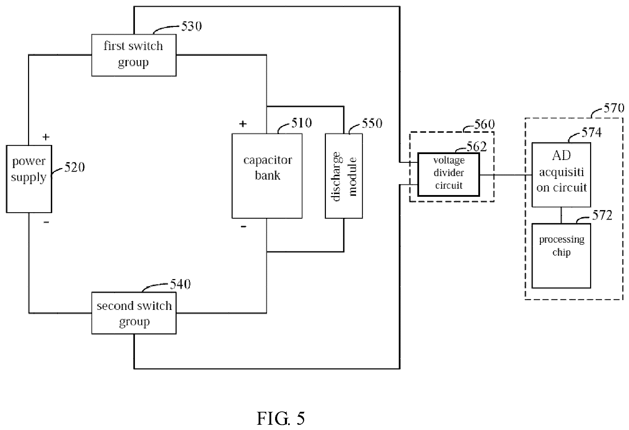

In an embodiment, as shown in FIG 5, a charge and discharge monitoring circuit is provided, which includes a controller 570, a capacitor bank 510, a power supply 520 configured to power the capacitor bank 510, a first switch group 530 connected between a positive electrode of the power supply 520 and a positive electrode of the capacitor bank 510, a second switch group 540 connected between a negative electrode of the power supply 520 and a negative electrode of the capacitor bank 510, a discharge module 550 connected between the positive electrode of the capacitor bank 510 and the negative electrode of the capacitor bank 510, a voltage detection circuit 560 connected between the first switch group 530 and the second switch group 540; the controller 570 is respectively connected to the voltage detection circuit 560, the first switch group 530, the second switch group 540 and the discharge module 550. The voltage detection circuit 560 includes a voltage divider circuit 562. A first detection terminal of the voltage divider circuit 562 is connected to the third switch interface of the first switch group 530; and a second detection terminal of the voltage divider circuit 562 is connected to the third switch interface of the second switch group 540, and an output terminal of the voltage divider circuit 562 is connected to the controller 570.

The voltage divider circuit 562 can consist of one voltage divider resistor, or consist of a plurality of voltage divider resistors in series. It should be noted that the resistance value of the voltage divider resistor can be calculated according to the requirements of acquisition parameters of a control device.

Specifically, the voltage divider circuit 562 is connected between the controller and the switch group (the first switch group and the second switch group), the voltage divider circuit 562 can perform voltage dividing on the received power supply signal to obtain the corresponding charge voltage signal, and then the controller 570 can acquire the charge voltage signal to obtain the charge voltage, thereby implementing the real-time monitoring of the charge voltage of the tumor therapy instrument. The voltage divider circuit 562 can perform the voltage dividing on the received discharge signal to obtain the corresponding discharge voltage signal, and then the controller 570 can acquire the discharge voltage signal to obtain the discharge voltage, thereby implementing the real-time monitoring of the discharge voltage of the tumor therapy instrument.

In an example, the voltage detection circuit may further include a filter circuit connected to the voltage divider circuit. The filter circuit may filter the charge voltage signal, and then obtain the filtered charge voltage signal, such that the charge voltage signal acquired by the controller is more accurate. The filter circuit can filter the discharge voltage signal, and then can obtain the filtered discharge voltage signal, such that the discharge voltage signal acquired by the controller is more accurate.

In the above-mentioned real-time charge and discharge monitoring circuit, the voltage divider circuit performs the voltage dividing on the voltage signals (the charge voltage signal and discharge voltage signal), so that the voltage signals (the charge voltage signal and discharge voltage signal) obtained after the voltage dividing can satisfy the requirements of the parameter acquisition of the controller and meanwhile the accuracy of the monitoring of the charge voltage is improved.

In a specific embodiment, as shown in FIG 5, the controller 570 includes a processing chip 572 and an AD acquisition circuit 574 connected to the processing chip 572.

The processing chip 572 can be, but not limited to, a single-chip microcomputer (SCM) system chip, an ARM processing chip, and an FPGA processing chip. The AD acquisition circuit 574 refers to an acquisition circuit that can perform analog-to-digital conversion on a signal.

Specifically, the processing chip 572 is connected to the AD acquisition circuit 574, and the AD acquisition circuit 574 is connected to the voltage detection circuit 560. The processing chip 572 can drive the AD acquisition circuit 574 to operate, and the AD acquisition circuit 574 can acquire the charge voltage signal outputted from the voltage detection circuit 560, and convert the analog charge voltage signal into a digital charge voltage through the analog-to-digital conversion processing, and transmit the digital charge voltage to the processing chip 572, thereby implementing the real-time monitoring of the charge voltage. The AD acquisition circuit 574 can also acquire the discharge voltage signal outputted from the voltage detection circuit 560, and convert the analog discharge voltage signal into the digital discharge voltage through the analog-to-digital conversion processing, and transmit the digital discharge voltage to the processing chip 572, thereby implementing the real-time monitoring of the discharge voltage.

In an example, the processing chip can transmit the charge voltage and the discharge voltage to an upper computer, and the charge voltage and the discharge voltage can be monitored in real time by the upper computer.

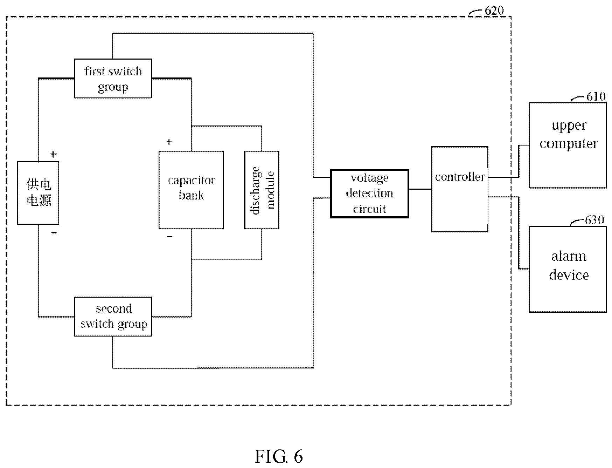

In an embodiment, as shown in FIG 6, a tumor therapy instrument is further provided, which includes an upper computer 640 and the charge and discharge monitoring circuit 620 in any one of the above-mentioned embodiments; and the upper computer 610 is connected to a controller.

The upper computer 610 may be, but is not limited to, a tablet computer and a personal computer (PC).

Specifically, the voltage detection circuit can detect the power supply signal outputted from the power supply to the capacitor bank, and obtain the charge voltage signal; the controller acquires the charge voltage signal, and then transmits the acquired charge voltage to the upper computer to implement the real-time monitoring of the charge voltage of the tumor therapy instrument. The voltage detection circuit can also detect the power supply signal outputted from the capacitor bank to the discharge module, and then obtain the discharge voltage signal. The controller acquires the discharge voltage signal, and then transmits the acquired discharge voltage to the upper computer to implement the real-time monitoring of the tumor therapy instrument. The circuit structure of the tumor therapy instrument is simple, which improves the real-time monitoring of the pulses.

Further, the upper computer 610 can also display a waveform diagram corresponding to the charge voltage and the charge current, so that the user can observe the corresponding waveform diagram, and control the tumor therapy instrument accordingly according to the waveform diagram.

In a specific embodiment, as shown in FIG 6, an alarm device 630 connected to the controller is further included.

The alarm device 630 may be a flashing light or a buzzer, or a combination of the flashing light and the buzzer.

Specifically, the controller can process the charge voltage and the discharge voltage. When the charge voltage is abnormal (for example, the charge voltage exceeds a safety threshold), the alarm device 630 is triggered, such that the alarm device 630 generates an alarm; when the discharge voltage is abnormal (such as the discharge voltage exceeds a safety threshold), the alarm device 630 is triggered, such that the alarm device 630 generates an alarm, thereby implementing a timely response to the monitoring situation of the tumor therapy instrument and enhancing the safety performance of the tumor therapy instrument.

The technical features of the above-mentioned embodiments can be combined arbitrarily. In order to make the description concise, all possible combinations of the various technical features in the above-mentioned embodiments are not described. However, as long as there is no contradiction in the combination of these technical features, all should be considered as the scope of the present invention if they fall within such a scope as defined by the appended claims.

The above-mentioned embodiments are merely some embodiments of the present invention, and their descriptions are more specific and detailed, but they should not be understood as limiting the scope of the present invention

The present invention is defined only by the appended claims.

## Claims

1. A charge and discharge monitoring circuit comprising a capacitor bank (110, 210, 310, 410, 510) and a power supply (120, 220, 320, 420, 520) configured to power the capacitor bank; further comprising:
a first switch group (130, 230, 330, 430, 530), wherein a first switch interface of the first switch group is connected to a positive electrode of the power supply, and a second switch interface of the first switch group is connected to a positive electrode of the capacitor bank;
a second switch group (140, 240, 340, 440, 540), wherein a first switch interface of the second switch group is connected to a negative electrode of the power supply, and a second switch interface of the second switch group is connected to a negative electrode of the capacitor bank;
a discharge module (150, 250, 350, 450, 550), wherein an input terminal of the discharge module is connected to the positive electrode of the capacitor bank, and an output terminal of the discharge module is connected to the negative electrode of the capacitor bank;
a voltage detection circuit (160, 260, 360, 460, 560), wherein a first detection terminal of the voltage detection circuit is connected to a third switch interface of the first switch group, and a second detection terminal of the voltage detection circuit is connected to a third switch interface of the second switch group, the voltage detection circuit is configured to monitor a charge voltage signal of the capacitor bank and monitor a discharge voltage signal of the discharge module;
a controller (170, 270, 370, 470, 570), wherein the controller is respectively connected to an output terminal of the voltage detection circuit, a control interface of the first switch group, a control interface of the second switch group and a control terminal of the discharge module; wherein the controller is configured to, when receiving a charge monitoring instruction, control the discharge module to turn off, and control the first switch group and the second switch group to turn on, and acquire a charge voltage signal to obtain a charge voltage; wherein the controller is configured to, when receiving a discharge monitoring instruction, control the discharge module to turn on, and control the first switch group and the second switch group to turn off, and acquire a discharge voltage signal to obtain a discharge voltage.

2. The charge and discharge monitoring circuit according to claim 1, wherein the discharge module (250) comprises a first switch device (252) and a discharge load (254);
a first end of the first switch device is connected to a first end of the discharge load, a second end of the first switch device is connected to one end of the capacitor bank (210), and
a third end of the first switch device is connected to the controller (270), a second end of the discharge load is connected to the other end of the capacitor bank.

3. The charge and discharge monitoring circuit according to claim 1, wherein the first switch group (330) comprises at least two second switch devices (332) connected in series.

4. The charge and discharge monitoring circuit according to claim 1, wherein the second switch group (340) comprises at least two third switch devices (342) connected in series.

5. The charge and discharge monitoring circuit according to claim 1, further comprising a first isolation module (480);
wherein a first input terminal of the first isolation module is connected to the first switch group, a second input terminal of the first isolation module is connected to the second switch group, and an output terminal of the first isolation module is connected to the voltage detection circuit.

6. The charge and discharge monitoring circuit according to claim 5, further comprising a second isolation module (490);
wherein the second isolation module is connected between the voltage detection circuit and the controller.

7. The charge and discharge monitoring circuit according to claim 1, wherein the voltage detection circuit (560) comprises a voltage divider circuit (562);
a first detection terminal of the voltage divider circuit is connected to the third switch interface of the first switch group, and a second detection terminal of the voltage divider circuit is connected to the third switch interface of the second switch group, and an output terminal of the voltage divider circuit is connected to the controller.

8. The charge and discharge monitoring circuit according to any one of claims 1 to 7, wherein the controller (570) comprises a processing chip (572) and an AD acquisition circuit (574) connected to the processing chip.

9. A tumor therapy instrument, **characterized by** comprising an upper computer (610), and the charge and discharge monitoring circuit according to any one of claims 1 to 8; wherein the upper computer is connected to the controller.

10. The tumor therapy instrument according to claim 9, further comprising an alarm device (630) connected to the controller.

## Patentansprüche

1. Ladungs- und Entladungsüberwachungsschaltung, umfassend eine Kondensatorbank (110, 210, 310, 410, 510) und eine Stromversorgung (120, 220, 320, 420, 520), die so konfiguriert ist, dass sie die Kondensatorbank mit Strom versorgt; ferner Folgendes umfassend:
eine erste Schaltergruppe (130, 230, 330, 430, 530),
wobei eine erste Schalterschnittstelle der ersten Schaltergruppe mit einer positiven Elektrode der Stromversorgung verbunden ist, und eine zweite Schalterschnittstelle der ersten Schaltergruppe mit einer positiven Elektrode der Kondensatorbank verbunden ist;
eine zweite Schaltergruppe (140, 240, 340, 440, 540),
wobei eine erste Schalterschnittstelle der zweiten Schaltergruppe mit einer negativen Elektrode der Stromversorgung verbunden ist, und eine zweite Schalterschnittstelle der zweiten Schaltergruppe mit einer negativen Elektrode der Kondensatorbank verbunden ist;
ein Entladungsmodul (150, 250, 350, 450, 550),
wobei ein Eingangsanschluss des Entladungsmoduls mit der positiven Elektrode der Kondensatorbank verbunden ist, und ein Ausgangsanschluss des Entladungsmoduls mit der negativen Elektrode der Kondensatorbank verbunden ist;
eine Spannungserkennungsschaltung (160, 260, 360, 460, 560),
wobei ein erster Erkennungsanschluss der Spannungserkennungsschaltung mit einer dritten Schalterschnittstelle der ersten Schaltergruppe verbunden ist, und ein zweiter Erkennungsanschluss der Spannungserkennungsschaltung mit einer dritten Schalterschnittstelle der zweiten Schaltergruppe verbunden ist, die Spannungserkennungsschaltung konfiguriert ist, um ein Ladungsspannungssignal der Kondensatorbank zu überwachen und ein Entladungsspannungssignal des Entladungsmoduls zu überwachen;
eine Steuerung (170, 270, 370, 470, 570),
wobei die Steuerung jeweils mit einem Ausgangsanschluss der Spannungserkennungsschaltung, einer Steuerschnittstelle der ersten Schaltergruppe, einer Steuerschnittstelle der zweiten Schaltergruppe und einem Steueranschluss des Entladungsmoduls verbunden ist; wobei die Steuerung konfiguriert ist, um, wenn sie einen Ladungsüberwachungsbefehl empfängt, das Entladungsmodul so zu steuern, dass es ausgeschaltet wird, und die erste Schaltergruppe und die zweite Schaltergruppe so zu steuern, dass sie eingeschaltet werden, und ein Ladungsspannungssignal zu erfassen, um eine Ladungsspannung zu erzielen; wobei die Steuerung konfiguriert ist, um, wenn sie einen Entladungsüberwachungsbefehl empfängt, das Entladungsmodul so zu steuern, dass es eingeschaltet wird, und die erste Schaltergruppe und die zweite Schaltergruppe so zu steuern, dass sie ausgeschaltet werden, und ein Entladungsspannungssignal zu erfassen, um eine Entladungsspannung zu erzielen.

2. Ladungs- und Entladungsüberwachungsschaltung nach Anspruch 1, wobei das Entladungsmodul (250) eine erste Schaltervorrichtung (252) und eine Entladungslast (254) umfasst;
ein erstes Ende der ersten Schaltervorrichtung mit einem ersten Ende der Entladungslast verbunden ist,
ein zweites Ende der ersten Schaltervorrichtung mit einem Ende der Kondensatorbank (210) verbunden ist, und
ein drittes Ende der ersten Schaltervorrichtung mit der Steuerung (270) verbunden ist,
ein zweites Ende der Entladungslast mit dem anderen Ende der Kondensatorbank verbunden ist.

3. Ladungs- und Entladungsüberwachungsschaltung nach Anspruch 1, wobei die erste Schaltergruppe (330) mindestens zwei in Reihe geschaltete zweite Schaltervorrichtungen (332) umfasst.

4. Ladungs- und Entladungsüberwachungsschaltung nach Anspruch 1, wobei die zweite Schaltergruppe (340) mindestens zwei in Reihe geschaltete dritte Schaltervorrichtungen (342) umfasst.

5. Ladungs- und Entladungsüberwachungsschaltung nach Anspruch 1, ferner umfassend ein erstes Isolationsmodul (480);
wobei ein erster Eingangsanschluss des ersten Isolationsmoduls mit der ersten Schaltergruppe verbunden ist, ein zweiter Eingangsanschluss des ersten Isolationsmoduls mit der zweiten Schaltergruppe verbunden ist, und ein Ausgangsanschluss des ersten Isolationsmoduls mit der Spannungserkennungsschaltung verbunden ist.

6. Ladungs- und Entladungsüberwachungsschaltung nach Anspruch 5, ferner umfassend ein zweites Isolationsmodul (490);
wobei das zweite Isolationsmodul zwischen der Spannungserkennungsschaltung und der Steuerung angeschlossen ist.

7. Ladungs- und Entladungsüberwachungsschaltung nach Anspruch 1, wobei die Spannungserkennungsschaltung (560) eine Spannungsteilerschaltung (562) umfasst;
ein erster Erkennungsanschluss der Spannungsteilerschaltung mit der dritten Schalterschnittstelle der ersten Schaltergruppe verbunden ist, und ein zweiter Erkennungsanschluss der Spannungsteilerschaltung mit der dritten Schalterschnittstelle der zweiten Schaltergruppe verbunden ist, und ein Ausgangsanschluss der Spannungsteilerschaltung mit der Steuerung verbunden ist.

8. Ladungs- und Entladungsüberwachungsschaltung nach einem der Ansprüche 1 bis 7, wobei die Steuerung (570) einen Verarbeitungschip (572) und eine mit dem Verarbeitungschip verbundene A/D-Erfassungsschaltung (574) umfasst.

9. Tumortherapieinstrument, **dadurch gekennzeichnet, dass** es einen oberen Computer (610) und die Ladungs- und Entladungsüberwachungsschaltung nach einem der Ansprüche 1 bis 8 umfasst;
wobei der obere Computer mit der Steuerung verbunden ist.

10. Tumortherapieinstrument nach Anspruch 9, ferner umfassend eine Alarmvorrichtung (630), die mit der Steuerung verbunden ist.

## Revendications

1. Circuit de surveillance de charge et de décharge comprenant une batterie de condensateurs (110, 210, 310, 410, 510) et une alimentation électrique (120, 220, 320, 420, 520) configurée pour alimenter la batterie de condensateurs ; comprenant en outre :
un premier groupe de commutateurs (130, 230, 330, 430, 530),
dans lequel une première interface de commutateurs du premier groupe de commutateurs est reliée à une électrode positive de l'alimentation électrique, et une deuxième interface de commutateurs du premier groupe de commutateurs est reliée à une électrode positive de la batterie de condensateurs ;
un deuxième groupe de commutateurs (140, 240, 340, 440, 540),
dans lequel une première interface de commutateurs du deuxième groupe de commutateurs est reliée à une électrode négative de l'alimentation électrique, et une deuxième interface de commutateurs du deuxième groupe de commutateurs est reliée à une électrode négative de la batterie de condensateurs ;
un module de décharge (150, 250, 350, 450, 550),
dans lequel une borne d'entrée du module de décharge est reliée à l'électrode positive de la batterie de condensateurs, et une borne de sortie du module de décharge est reliée à l'électrode négative de la batterie de condensateurs ;
un circuit de détection de tension (160, 260, 360, 460, 560),
dans lequel une première borne de détection du circuit de détection de tension est reliée à une troisième interface de commutateurs du premier groupe de commutateurs, et une deuxième borne de détection du circuit de détection de tension est reliée à une troisième interface de commutateurs du deuxième groupe de commutateurs, le circuit de détection de tension est configuré pour surveiller un signal de tension de charge de la batterie de condensateurs et surveiller un signal de tension de décharge du module de décharge ;
un dispositif de commande (170, 270, 370, 470, 570),
dans lequel le dispositif de commande est respectivement relié à une borne de sortie du circuit de détection de tension, une interface de commande du premier groupe de commutateurs, une interface de commande du deuxième groupe de commutateurs et une borne de commande du module de décharge ; dans lequel le dispositif de commande est configuré, lors de la réception d'une instruction de surveillance de charge, pour commander la désactivation du module de décharge, et commander l'activation du premier groupe de commutateurs et du deuxième groupe de commutateurs, et acquérir un signal de tension de charge pour obtenir une tension de charge ; dans lequel le dispositif de commande est configuré, lors de la réception d'une instruction de surveillance de décharge, pour commander l'activation du module de décharge, et commander la désactivation du premier groupe de commutateurs et du deuxième groupe de commutateurs, et acquérir un signal de tension de décharge pour obtenir une tension de décharge.

2. Circuit de surveillance de charge et de décharge selon la revendication 1, dans lequel le module de décharge (250) comprend un premier dispositif de commutateurs (252) et une charge de décharge (254) ;
une première extrémité du premier dispositif de commutateurs est reliée à une première extrémité de la charge de décharge,
une deuxième extrémité du premier dispositif de commutateurs est reliée à une extrémité de la batterie de condensateurs (210), et
une troisième extrémité du premier dispositif de commutateurs est reliée au dispositif de commande (270),
une deuxième extrémité de la charge de décharge est reliée à l'autre extrémité de la batterie de condensateurs.

3. Circuit de surveillance de charge et de décharge selon la revendication 1, dans lequel le premier groupe de commutateurs (330) comprend au moins deux deuxièmes dispositifs de commutateurs (332) reliés en série.

4. Circuit de surveillance de charge et de décharge selon la revendication 1, dans lequel le deuxième groupe de commutateurs (340) comprend au moins deux troisièmes dispositifs de commutateurs (342) reliés en série.

5. Circuit de surveillance de charge et de décharge selon la revendication 1, comprenant en outre un premier module d'isolation (480) ;
dans lequel une première borne d'entrée du premier module d'isolation est reliée au premier groupe de commutateurs, une deuxième borne d'entrée du premier module d'isolation est reliée au deuxième groupe de commutateurs, et une borne de sortie du premier module d'isolation est reliée au circuit de détection de tension.

6. Circuit de surveillance de charge et de décharge selon la revendication 5, comprenant en outre un deuxième module d'isolation (490) ;
dans lequel le deuxième module d'isolation est relié entre le circuit de détection de tension et le dispositif de commande.

7. Circuit de surveillance de charge et de décharge selon la revendication 1, dans lequel le circuit de détection de tension (560) comprend un circuit diviseur de tension (562) ;
une première borne de détection du circuit diviseur de tension est reliée à la troisième interface de commutateurs du premier groupe de commutateurs, et une deuxième borne de détection du circuit diviseur de tension est reliée à la troisième interface de commutateurs du deuxième groupe de commutateurs, et une borne de sortie du circuit diviseur de tension est reliée au dispositif de commande.

8. Circuit de surveillance de charge et de décharge selon l'une quelconque des revendications 1 à 7, dans lequel le dispositif de commande (570) comprend une puce de traitement (572) et un circuit d'acquisition A/N (574) relié à la puce de traitement.

9. Instrument de thérapie antitumorale, **caractérisé en ce qu'**il comprend un ordinateur supérieur (610), et le circuit de surveillance de charge et de décharge selon l'une quelconque des revendications 1 à 8 ;
dans lequel l'ordinateur supérieur est relié au dispositif de commande.

10. Instrument de thérapie antitumorale selon la revendication 9, comprenant en outre un dispositif d'alarme (630) relié au dispositif de commande.
